# EUROPEAN PATENT SPECIFICATION

(11) **EP 1 969 573 B1**
(45) Date of publication and mention of the grant of the patent: **04.11.2009**
(21) Application number: 06831502.7
(22) Date of filing: 28.12.2006
(51) Int. Cl.: G08B 21/04

(54) **PERSONAL WARNING APPARATUS**
PERSÖNLICHE WARNVORRICHTUNG
APPAREIL D'AVERTISSEMENT PERSONNEL

(30) Priority: 28.12.2005 GB 0526478; 28.10.2006 GB 0621500
(43) Date of publication of application: 17.09.2008
(73) Proprietor: CradleSafe Limited, Glasgow G75 8SU (GB)
(72) Inventor: SOMMERVILLE, Paul, Richard, Glasgow G75 8SU (GB)
(74) Representative: D'Arcy, Julia
(86) International application number: PCT/GB2006/004951
(87) International publication number: WO 2007/074345

(56) References cited:
- WO-A-02/41277
- WO-A-2004/043263
- GB-A- 2 396 043
- US-A- 6 054 926
- US-A1- 2002 057 202
- US-B1- 6 450 168
- US-B1- 6 975 230

## Description

The present invention relates to a personal warning apparatus. In particular, the present invention relates to an apparatus and method for detecting and preventing the onset of Sudden Infant Death Syndrome. The present invention also relates to an apparatus and method for detecting and preventing local environmental conditions associated with Sudden Infant Death Syndrome.

Sudden Infant Death Syndrome (SIDS) or "cot death" is known to primarily affect infants aged between 3 and 9 months. SIDS occurs unexpectedly and quickly to apparently healthy infants, usually during periods of sleep. There remains some debate as to the causative factors in SIDS, although it is established that ensuring that the infant's breathing remains unobstructed can help to reduce the risk. In addition, it has been proposed that preventing the infant from becoming too hot, and ensuring that the infant sleeps on its back, can help to prevent the risk of SIDS.

At present, several different types of apparatus and methods exist for detecting SIDS.. However, the existing apparatus and methods have several disadvantages which restrict their effectiveness and ease of use. For example, many existing SIDS detection systems use detectors that sense movement of the infant. However, many of these detection systems are triggered by minor movements or when the infant stops moving for only a short period of time, creating many false alarms. Furthermore, several of these motion detectors comprise a single unit which is attached to the infant. These motion detection units have no fixed point of reference and are therefore more susceptible to false alarms.

Other existing apparatus for detecting SIDS measure the breathing pattern, the temperature, the heartbeat or other vital signs of the infant (some of these devices are known as apnea alarms). However, as infants' heartbeats and breathing patterns are often irregular, such devices can generate false alarms.

US-B1-6975230 discloses a personal warning apparatus according to the preamble of claim 1.

It can be seen that existing SIDS detection systems are prone to causing false alarms, and provide no way of preventing the initial onset of SIDS. It is of particular importance to develop a SIDS detection system that does not produce false alarms, as these can prove stressful and time consuming for the infant's carer. Moreover, numerous false alarms lessen the impact of the alarm on the carer, such that the carer is more likely to inadvertently neglect a genuine alarm. In addition several prior art systems, such as apnea alarms, detect SIDS rather than the causative factors. As such, these alarms provide no way of pre-empting or preventing SIDS.

Therefore, it is an object of the present invention to overcome at least some of the drawbacks associated with the prior art.

A further object of the invention is to provide an apparatus and method for detecting the onset of SIDS.

A still further object of the invention is to provide an apparatus and method for preventing the onset of SIDS.

Further aims and objects of the invention will become apparent from a reading of the following description.

According to the present invention there is provided a personal warning apparatus comprising at least one relative motion detector adapted to be positioned on or around the body of an individual, an evaluation module having a predetermined activation threshold, and at least one communication means, the at least one relative motion detector being operatively linked to the evaluation module, and the evaluation module being operatively linked to the at least one communication means, wherein the evaluation module responsive to an evaluation is able to send an alarm signal to the at least one communication means,
wherein the relative motion detector is adapted to detect lateral movement where lateral movement is in the direction of the longest axis of the individual's body.

Preferably the evaluation module is adapted to sense motion indicative of the onset of Sudden Infant Death Syndrome.

The evaluation module can be adapted to activate the at least one communication means on sensing conditions associated with the onset of Sudden Infant Death Syndrome.

The evaluation module can be a suitably programmed processor. The processor can be configured to evaluate detected motion.

Optionally the processor is a microprocessor.

Alternatively the processor is an application specific integrated circuit.

Preferably the apparatus further comprises a detector unit adapted to be positioned on or around the body of an individual.

Optionally, the apparatus further comprises an alarm unit remote from the detector unit, the detector unit being operatively connected to the alarm unit.

Preferably the apparatus further comprises a relay unit remote from the alarm unit and the detector unit, the detector unit being operatively connected to the relay unit, and the relay unit being operatively connected to the alarm unit.

Optionally the detector unit comprises the at least one relative motion detector, the evaluation module and at least one communication means.

Alternatively the detector unit comprises the at least one relative motion detector and at least one communication means, and the alarm unit comprises at least one communication means, the evaluation module and an alarm, the evaluation module being operatively connected to the alarm.

Preferably the detector unit comprises the at least one relative motion detector and at least one communication means, and the relay unit comprises at least one communication means and the evaluation module, and the alarm unit comprises at least one communication means and an alarm, the at least one communication means in the alarm unit being operatively connected to the alarm.

### Optionally the predetermined activation threshold is adjustable.

The relative motion detection means can be adapted to further detect rotational movement, with respect to the longest axis of the individual's body. Therefore, this can be used to monitor whether the infant has moved onto their side.

The relative motion detection means can be adapted to further detect vertical movement. When an individual is lying down the vertical movement is with respect to the shortest axis of their body. Therefore, this can be used to monitor the individual's breathing.

The present invention will now be described by way of example only, with reference to the accompanying drawings in which:
Figure 1 is a representation of one embodiment of the SIDS alarm apparatus in accordance with the present invention; and
Figure 2 is a representation of a SIDS alarm apparatus containing a sleeping sack in accordance with an example.

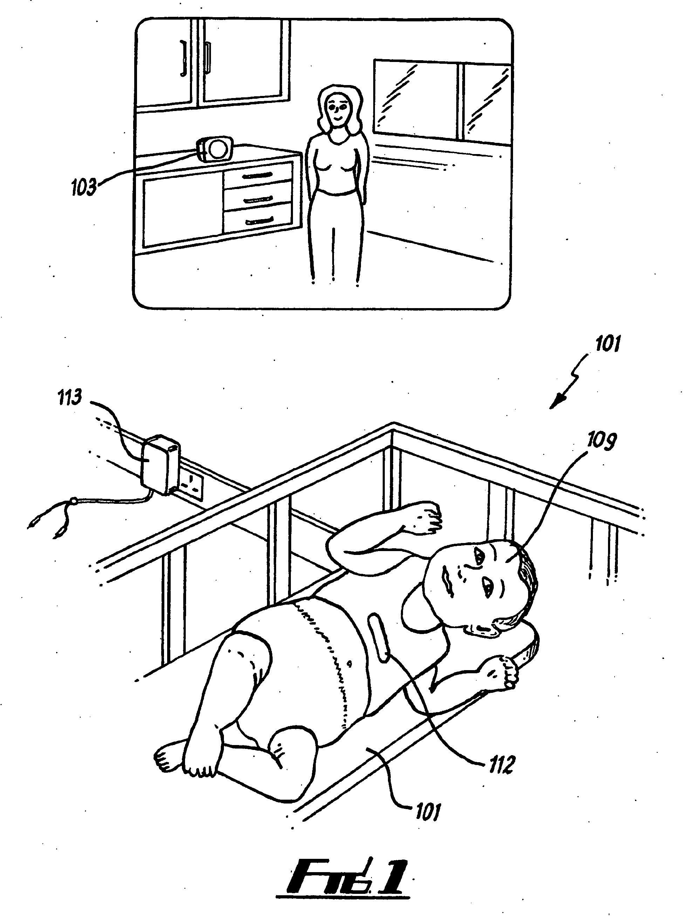

Referring to Figure 1 the SIDS prevention and detection apparatus is generally depicted at 101 and is comprised of a detector unit 112, a signal repeater or relay unit 113 and alarm unit 103. The detector unit 112 contains a radio-frequency transmitter microchip (not shown) and communicates with the relay unit 113 using radio frequency signals. The detector unit 112 contains a triaxial accelerometer (not shown) which measures rotational, vertical and lateral movement. The detector unit 112 is encased in a liquid resistant pouch (not shown). The pouch is secured to the chest of an infant 109, at rest in a cot 110. The detector unit 112 also contains a temperature sensor (not shown) to monitor the infant's temperature. The relay unit 113 contains a radio-frequency transceiver microchip (not shown) and communicates with the alarm unit 103, which contains a radio-frequency receiver (not shown), using radio-frequency signals. The relay unit also contains a microprocessor which acts as an evaluation module. The microprocessor evaluates the accelerometer readings and determines whether or not an alarm should be generated. If an accelerometer reading is evaluated as high risk, the microprocessor sends an alarm signal to the radio-frequency transceiver microchip which communicates with the alarm unit 103, which in turn generates an alarm.

The evaluation module can be a suitably programmed processor, microprocessor or application specific integrated circuit, configured to evaluate detected motion and/or temperature.

The evaluation module is responsive to the evaluation performed. If it concludes that a signal, or set of signals, is associated with risk to the individual it will send an alarm signal, by way of an electrical signal, to the radio-frequency transceiver. The radio frequency transceiver will transmit a signal to the alarm unit which will then raise an alarm. As the evaluation module is programmable and adjustable, it can be set to have predetermined movement and/or temperature thresholds, which mitigates the generation of false alarms. These thresholds are activation thresholds beyond which the evaluation module will conclude that the infant is at risk. In particular, the evaluation module can be programmed such that it activates the radio-frequency transceiver, and generates an alarm, on sensing movement and/or temperature conditions associated with the onset of SIDS.

In the example described, the components of the detector unit are linked by way of a printed circuit board and the detector unit is powered by a battery. The components of the relay unit are also linked using a printed circuit board, and the relay unit is mains electricity powered. Similarly, the components of the alarm unit are linked using a printed circuit board and the alarm unit is mains electricity powered. The detector unit, relay unit and alarm unit are linked by radio-frequency transmitters/receivers or transceivers and thus are operatively connected.

The evaluation module is adapted to be sensitive to motion indicative of the onset of Sudden Infant Death Syndrome.

The detector unit can be removed from the pouch so that the pouch can be washed. The liquid resistant material prevents the ingress of liquid into the pouch and therefore protects the detector and transmitter equipment. In this example the pouch containing the detector unit is secured against the infant's chest using an adjustable strap. In an alternative embodiment the temperature sensor is located in the pouch.

The apparatus can contain one or more temperature sensors that measure one or both of the infant's temperature and the ambient temperature.

The detector can be secured against the infant's chest by any suitable means, including for example a harness or hook and loop fasteners. In one embodiment, the detector unit is placed in a pouch in a baby vest comprising wicking fabric, a padded chest area and hook and loop fastenings. The detector unit can be removed from the infant's clothing for washing.

In an alternative embodiment the detector unit is integral to the infant's clothing.

In use, the detector unit communicates the movements of the infant with the relay unit using small, low-powered digital radio transceivers. The same technology is used to communicate between the relay unit and the alarm unit.

In a further alternative embodiment, the detector unit communicates directly with the alarm unit, and there is no relay unit present. In this embodiment the detector unit also comprises the evaluation module which works in the normal way, sending an alarm signal to the alarm unit in the event of a high risk condition being assessed. Alternatively, the evaluation module is placed in the alarm unit rather then the detector unit. The detector unit is in constant communication with the alarm unit. If the evaluation module detects high risk conditions an alarm is generated.

In a still further embodiment, the detector unit further comprises the evaluation module and an alarm, which acts as a communication means. In this particular embodiment it is not necessary for the detector unit to contain a radio-frequency transceiver, although it may optionally do so. The detector unit may act alone to detect, evaluate a high risk condition, and create an audible and/or visible alarm.

Accelerometer devices measure the acceleration and therefore movement in one or more directions. In this example one triaxial accelerometer capable of measuring in all three x, y and z axes is used. It is also feasible that two biaxial accelerometers both measuring in two axes could instead be used, as could three uniaxial accelerometers. Monitoring movement in one axis confirms that the infant is breathing. Detecting when the infant moves onto its side is possible by monitoring movement in another axis. According to the invention, significant movement in the remaining axis detects when the infant moves up or down the cot, possibly under their blanket.

In the example described the accelerometer acts as a relative motion detector and the radio-frequency transmitters/receivers in the detector unit, the relay unit, and the alarm unit, act as communication means.

When the accelerometer detects conditions associated with the onset of SIDS, it sends an electrical signal to the microprocessor which acts as an evaluation module. If the evaluation module senses that the infant is at risk, it will send an electrical signal to the radio-frequency transmitter which in turn transmits a radio-frequency signal to the relay unit which acts as an intermediate transceiver unit. The relay unit transmits a radio-frequency signal to the alarm unit which acts as a remote alert generating means, and the alarm unit generates an audible and/or visible alarm to indicate to a carer that the infant is in a high risk state.

The accelerometer can detect rotational movement. The rotational movement is with respect to the longest axis of an infant's body. That is, with respect to the infant's height. Therefore, this can be used to monitor whether the infant has moved onto their side. In use, the rotational movement threshold can be set to between 30° and 45°.

One way of performing this measurement is to measure the acceleration readings on three separate axes to determine their orientation. As the accelerometers are effectively measuring an effect of gravity, an axis in line with the ground will give a reading of g. The same axis oriented at 45° will give a reading of 0.5g. Thus, the orientation of the infant can be detected.

The accelerometer can also detect vertical movement. When an infant is lying down the vertical movement is with respect to the shortest axis of its body. This can be used to monitor the infant's breathing by, for example, averaging a number of acceleration readings (generated by the movement of the infant's chest,'i.e. breathing) and determining if the readings have varied by a particular threshold amount. If the readings do not vary by this amount, the device detects that the infant has stopped breathing.

According to the invention, the accelerometer detects lateral movement. The lateral movement is in the direction of the longest axis of the infant's body. That is with respect to the infant's height. This detection requires the use of high accuracy, fast sampling and low noise. The accelerometer provides a measure of the acceleration at a particular sampling instant and thus is used to indirectly measure distance moved in a particular direction. Therefore, this is used to monitor whether the infant has slid underneath any blankets or bed clothing.

An advantage of the present invention is that the evaluation module used is adjusted to have predetermined activation thresholds, i.e., varying the sensitivity levels. The thresholds can be preset during manufacture in accordance to the average, safe movements of an infant during sleep. In an alternative embodiment the thresholds can be adjusted by the infant's carer in accordance with individual sleeping habits. This requires apparatus with which the carer can make the necessary adjustments. For example, using suitable software and an existing computer the thresholds can be adjusted and the infant monitored. The same software can be used to display the temperature of the infant and to raise an alarm.

Alternatively, the thresholds of the evaluation unit can be adjusted using dials or the like attached to the detector unit, the relay unit and/or the alarm unit. Such adjustments can be made manually and without the requirement for a personal computer.

This is particularly useful, for example, for avoiding false alarms when an infant enters deep sleep. In deep sleep, an infant's breathing is more likely to be irregular or shallow, and thus it is useful to adjust the sensitivity of the evaluation of the accelerometer that monitors breathing.

The alarm can be activated via a radio-frequency transceiver or can be activated directly by the microprocessor. The microprocessor has preset and optionally programmable thresholds associated with the movement of the accelerometers. These thresholds are selected to mitigate the generation of false alarms.

Also in use, the movement of the infant may be partially restricted using, for example, a sleeping sack. In addition, the infant's temperature and/or ambient temperature may be monitored by temperature sensors and optionally evaluated by the microprocessor.

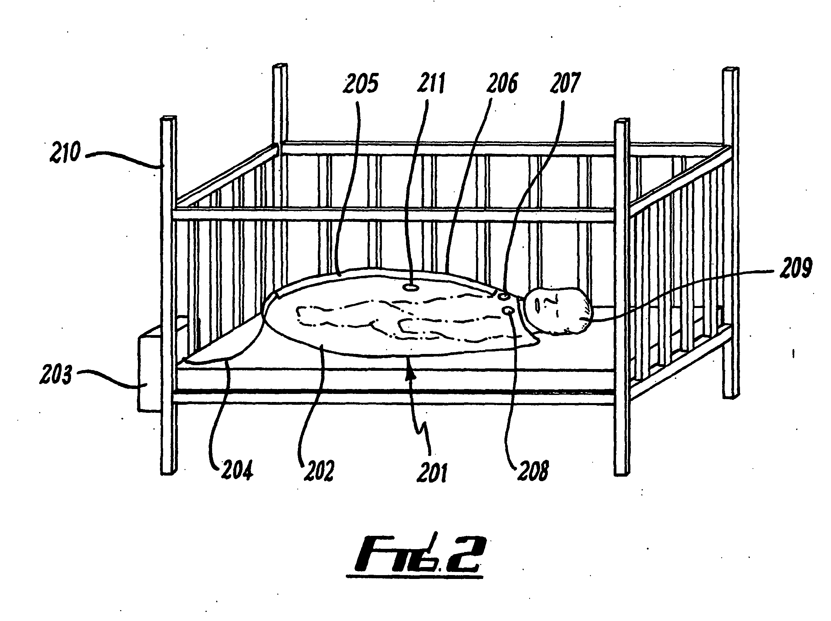

Figure 2 is a representation of a SIDS alarm apparatus in accordance with an example.

Referring to Figure 2 the SIDS prevention and detection apparatus is generally depicted at 201 and is comprised of a sack 202 and an alarm 203. The sack 202 is connected to the alarm 203 via wiring 204. Further wiring (not shown) is encased in a removable conduit 205 which slots into a channel 206 in the sack 202. The sack 202 comprises a magnetic switch 207 that is associated with a magnet 208 which is attached to an infant 209 at rest in a cot 210. The sack 202 further comprises a temperature sensor 211 for monitoring the infant's temperature. The temperature sensor 211 and the magnetic switch 207 are connected to the wiring (not shown) encased in the removable conduit 205.

The removable conduit 205 provides that all wiring for the alarm 203, the magnetic switch 207 and the temperature sensor 211 is isolated from the infant 209. This ensures the safety of the infant 209 and makes the sack 202 more comfortable for the infant 209 to sleep in. Consequently, the infant 209 is less restless during seep. The removable conduit 205 is made from a liquid resistant material to prevent ingress of liquids. The removable conduit 205 can be separated from the sack 202, which is particularly useful for the purposes of washing the sack 202. In this example the removable conduit 205 attaches to the channel 206 in the sack 205 by way of a hook and loop fastening system.

In the example illustrated in Figure 2, the magnetic switch and associated magnet act as a relative motion detector with an activation threshold that can be predetermined. This predetermined activation threshold can be preset or may be adjustable such that a user can set the threshold to their desired level.

In an alternative example the wiring is integral to the sack and does not run in a removable channel. In this alternative example, the wiring may still be removed from the sack for the purposes of washing or otherwise. The sack may itself provide a degree of water resistance.

In the example shown in Figure 2 the alarm 203 is connected to the sack 202 via wiring 204 and the sack 202 contains further wiring (not shown) for the temperature sensor 211 and the magnetic switch 207. The magnetic switch 207 is fixed in position, stitched into the lining of the sack 202.

In an alternative example, the wiring is replaced by remote transceivers (such as radio frequency transmitters and radio frequency receivers) such that the temperature sensor and the magnetic switch can communicate wirelessly with the alarm.

Again referring to Figure 2, the alarm 203 is an audible and visual alarm which is attached to the cot 210. In an alternative example, the apparatus communicates with a remote device using means such as radio frequency communication, such that a remote alarm is triggered.

The remote alarm may be placed next to a carer who is remote from the infant.

Referring once more to Figure 2, in use the infant 209 is fitted with a magnet 208 on its chest and is placed on its back in the sack 202. The sack 202 fits securely around the infant 209 such that minor movements are not constrained, but such that larger movements, for example turning in the sack 202 or sliding inside the sack 202, are prevented. This affords the infant 209 a more comfortable and safer sleeping environment.

The magnetic switch 207 is adjusted to a predetermined activation threshold such that if the infant 209 moves as part of the normal sleeping process the magnet 208 and magnetic switch 207 remain associated and the magnetic switch 207 is not triggered. However, should the sack 202 fail to constrain the infant 209 and allow the infant 209 to move into a "high risk" position (i.e. if the infant 209 slides inside the sack 202 or turns onto its front), the association between the magnet 208 and the magnetic switch 207 is broken, triggering the alarm 203. In this example, the predetermined activation threshold can be adjusted. In an alternative example, the predetermined activation threshold is not adjustable.

Once more referring to Figure 2, the magnetic switch 207 and magnet 208 remain associated when they stay within a certain distance of each other as set by the predetermined activation threshold. That is, the two devices will tolerate a certain degree of relative movement without becoming dissociated. As long as the magnetic switch 207 and the magnet 208 are associated, the magnetic switch 207 will not be activated and the alarm 203 will not be triggered. This affords the present apparatus a degree of flexibility in that the infant 209 may make "safe" movements without triggering the alarm 203. Therefore, the present apparatus prevents false alarms from occurring.

In the example described, the magnetic switch and the magnet act as a relative motion sensor that has a degree of tolerance to minor movements.

Referring still to Figure 2, in use the temperature sensor 211 affixed to the sack 202 continuously monitors the temperature of the infant 209. If the temperature deviates from the normal accepted temperature range, the alarm 203 is triggered. The alarm 203 may be set to produce a different type of alert depending on whether the temperature sensor 211 or the magnetic switch 207 triggers the alarm 203. For example, the alarm 203 may be an audible alert of different pitch for the temperature sensor 211 and the magnetic switch 207.

In a further example the temperature sensor is attached to the infant's abdomen. If the infant's temperature deviates out with the normal accepted temperature range, the temperature sensor will trigger the alarm. The temperature sensor can be adapted to be positioned on or around the infant or their clothing.

An advantage of the present invention is that it detects any movement by an infant into a high risk position. As such, the alarm is triggered before the infant stops breathing and before SIDS occurs. That is to say, the invention detects high risk environmental conditions associated with the onset of SIDS (by means of both the movement and the temperature sensor) rather than SIDS. Therefore, the present invention pre-empts SIDS and can allow a carer to attend to an infant in a high risk environment, before the infant stops breathing, increasing the chances of preventing SIDS.

Prior art SIDS alarms serve only to inform a carer that SIDS has occurred. As such, they do not afford the carer the opportunity to intervene, so that they might prevent SIDS from occurring. Furthermore, existing SIDS alarms do not combine a SIDS warning system with an apparatus for preventing an infant from adopting wan orientation associated with a higher risk of SIDS. In addition, existing SIDS alarms are prone to false alarms, which detract from their impact in the event of a genuine SIDS incident.

Improvements and modifications may be incorporated herein without deviating from the scope of the invention, as defined by the appended claims.

## Claims

1. A personal warning apparatus (101) comprising at least one relative motion detector adapted to be positioned on or around the body of an individual, an evaluation module having a predetermined activation threshold, and at least one communication means, the at least one relative motion detector being operatively linked to the evaluation module, and the evaluation module being operatively linked to the at least one communication means, wherein the evaluation module responsive to an evaluation is able to send an alarm signal to the at least one communication means, **characterised in that** the relative motion detector is adapted to detect lateral movement where lateral movement is in the direction of the longest axis of the individual's body.

2. An apparatus as claimed in claim 1, wherein the evaluation module is adapted to sense motion indicative of the onset of Sudden Infant Death Syndrome.

3. An apparatus as claimed in claims 1 or 2, wherein the evaluation module is adapted to activate the at least one communication means on sensing conditions associated with the onset of Sudden Infant Death Syndrome.

4. An apparatus as claimed in any preceding claim, wherein the evaluation module is a suitably programmed processor.

5. An apparatus as claimed in claim 4, wherein the processor is configured to evaluate detected motion.

6. An apparatus as claimed in claims 4 or 5, wherein the processor is a microprocessor or application specific integrated circuit.

7. An apparatus as claimed in any preceding claim wherein the apparatus further comprises a detector unit (112) adapted to be positioned on or around the body of an individual.

8. An apparatus as claimed in claim 7 wherein the apparatus further comprises an alarm unit (103) remote from the detector unit (112), the detector unit (112) being operatively connected to the alarm unit (103).

9. An apparatus as claimed in claim 8 wherein the apparatus further comprises a relay unit (113) remote from the alarm unit (103) and the detector unit (112) the detector unit (112) being operatively connected to the relay unit (113), and the relay unit (113) being operatively connected to the alarm unit (103).

10. An apparatus as claimed in claim 7 wherein the detector unit (112) comprises the at least one relative motion detector, the evaluation module and at least one communication means.

11. An apparatus as claimed in claim 8 wherein the detector unit (112) comprises the at least one relative motion detector and at least one communication means, and the alarm unit (103) comprises at least one communication means, the evaluation module and an alarm, the evaluation module being operatively connected to the alarm.

12. An apparatus as claimed in claim 9 wherein the detector unit (112) comprises the at least one relative motion detector and at least one communication means, and the relay unit (113) comprises at least one communication means and the evaluation module, and the alarm unit (103) comprises at least one communication means and an alarm, the at least one communication means in the alarm unit (103) being operatively connected to the alarm.

13. An apparatus as claimed in any preceding claim, wherein the predetermined activation threshold is adjustable.

14. An apparatus as claimed in any preceding claim, wherein the at least one relative motion detector is adapted to further detect rotational movement with respect to the longest axis of the individual's body.

15. An apparatus as claimed in any preceding claim, wherein the relative motion detector is adapted to further detect vertical movement with respect to the shortest axis of the individual's body.

## Patentansprüche

1. Eine persönliche Warnvorrichtung (101), die mindestens einen Detektor für relative Bewegung beinhaltet, der angepasst ist, um auf oder um den Körper einer Einzelperson positioniert zu werden, ein Evaluierungsmodul, das eine vorbestimmte Aktivierungsschwelle aufweist, und mindestens ein Kommunikationsmittel, wobei der mindestens eine Detektor für relative Bewegung operativ mit dem Evaluierungsmodul verknüpft ist und das Evaluierungsmodul operativ mit dem mindestens einen Kommunikationsmittel verknüpft ist, wobei das auf eine Evaluierung reagierende Evaluierungsmodul dazu fähig ist, ein Alarmsignal an das mindestens eine Kommunikationsmittel zu senden, **dadurch gekennzeichnet, dass** der Detektor für relative Bewegung angepasst ist, um eine laterale Bewegung zu erfassen, wobei laterale Bewegung in der Richtung der längsten Achse des Körpers der Einzelperson stattfindet.

2. Vorrichtung gemäß Anspruch 1, wobei das Evaluierungsmodul angepasst ist, um Bewegung zu erkennen, die indikativ für den Beginn des plötzlichen Kindstods ist.

3. Vorrichtung gemäß Anspruch 1 oder 2, wobei das Evaluierungsmodul angepasst ist, um das mindestens eine Kommunikationsmittel beim Erkennen von mit dem Beginn des plötzlichen Kindstods assoziierten Bedingungen zu aktivieren.

4. Vorrichtung gemäß einem der vorhergehenden Ansprüche, wobei das Evaluierungsmodul ein entsprechend passend programmierter Prozessor ist.

5. Vorrichtung gemäß Anspruch 4, wobei der Prozessor konfiguriert ist, um eine erfasste Bewegung zu evaluieren.

6. Vorrichtung gemäß Anspruch 4 oder 5, wobei der Prozessor ein Mikroprozessor oder eine anwendungsspezifische integrierte Schaltung ist.

7. Vorrichtung gemäß einem der vorhergehenden Ansprüche, wobei die Vorrichtung ferner eine Detektoreinheit (112) beinhaltet, die angepasst ist, um auf oder um den Körper einer Einzelperson positioniert zu werden.

8. Vorrichtung gemäß Anspruch 7, wobei die Vorrichtung ferner eine von der Detektoreinheit (112) abgelegene Alarmeinheit (103) beinhaltet, wobei die Detektoreinheit (112) operativ mit der Alarmeinheit (103) verbunden ist.

9. Vorrichtung gemäß Anspruch 8, wobei die Vorrichtung ferner eine von der Alarmeinheit (103) und der Detektoreinheit (112) abgelegene Relaiseinheit (113) beinhaltet, wobei die Detektoreinheit (112) operativ mit der Relaiseinheit (113) verbunden ist und die Relaiseinheit (113) operativ mit der Alarmeinheit (103) verbunden ist.

10. Vorrichtung gemäß Anspruch 7, wobei die Detektoreinheit (112) den mindestens einen Detektor für relative Bewegung, das Evaluierungsmodul und mindestens ein Kommunikationsmittel beinhaltet.

11. Vorrichtung gemäß Anspruch 8, wobei die Detektoreinheit (112) den mindestens einen Detektor für relative Bewegung und mindestens ein Kommunikationsmittel beinhaltet und die Alarmeinheit (103) mindestens ein Kommunikationsmittel, das Evaluierungsmodul und einen Alarm beinhaltet, wobei das Evaluierungsmodul operativ mit dem Alarm verbunden ist.

12. Vorrichtung gemäß Anspruch 9, wobei die Detektoreinheit (112) den mindestens einen Detektor für relative Bewegung und mindestens ein Kommunikationsmittel beinhaltet und die Relaiseinheit (113) mindestens ein Kommunikationsmittel und das Evaluierungsmodul beinhaltet und die Alarmeinheit (103) mindestens ein Kommunikationsmittel und einen Alarm beinhaltet, wobei das mindestens eine Kommunikationsmittel in der Alarmeinheit (103) operativ mit dem Alarm verbunden ist.

13. Vorrichtung gemäß einem der vorhergehenden Ansprüche, wobei die vorbestimmte Aktivierungsschwelle einstellbar ist.

14. Vorrichtung gemäß einem der vorhergehenden Ansprüche, wobei der mindestens eine Detektor für relative Bewegung angepasst ist, um ferner eine Drehbewegung bezüglich der längsten Achse des Körpers der Einzelperson zu erfassen.

15. Vorrichtung gemäß einem der vorhergehenden Ansprüche, wobei der Detektor für relative Bewegung angepasst ist, um ferner eine vertikale Bewegung bezüglich der kürzesten Achse des Körpers der Einzelperson zu erfassen.

## Revendications

1. Un appareil d'avertissement personnel (101) comprenant au moins un détecteur de mouvement relatif adapté pour être positionné sur ou autour du corps d'un individu, un module d'évaluation présentant un seuil d'activation prédéterminé, et au moins un moyen de communication, cet au moins un détecteur de mouvement relatif étant relié en fonctionnement au module d'évaluation, et le module d'évaluation étant relié en fonctionnement à cet au moins un moyen de communication, dans lequel le module d'évaluation sensible à une évaluation est à même d'envoyer un signal d'alarme à cet au moins un moyen de communication, **caractérisé en ce que** le détecteur de mouvement relatif est adapté pour détecter un déplacement latéral lorsque le déplacement latéral se fait dans le sens de l'axe le plus long du corps de l'individu.

2. Un appareil tel que revendiqué dans la revendication 1, dans lequel le module d'évaluation est adapté pour capter un mouvement indiquant le début du Syndrome de mort subite du nourrisson.

3. Un appareil tel que revendiqué dans les revendications 1 ou 2, dans lequel le module d'évaluation est adapté pour activer cet au moins un moyen de communication lorsqu'il capte des conditions associées au début du Syndrome de mort subite du nourrisson.

4. Un appareil tel que revendiqué dans n'importe quelle revendication précédente, dans lequel le module d'évaluation est un processeur programmé de façon appropriée.

5. Un appareil tel que revendiqué dans la revendication 4, dans lequel le processeur est configuré pour évaluer un mouvement détecté.

6. Un appareil tel que revendiqué dans les revendications 4 ou 5, dans lequel le processeur est un microprocesseur ou un circuit intégré spécifique.

7. Un appareil tel que revendiqué dans n'importe quelle revendication précédente dans lequel l'appareil comprend en outre une unité formant détecteur (112) adaptée pour être positionnée sur ou autour du corps d'un individu.

8. Un appareil tel que revendiqué dans la revendication 7 dans lequel l'appareil comprend en outre une unité formant alarme (103) distante de l'unité formant détecteur (112), l'unité formant détecteur (112) étant connectée en fonctionnement à l'unité formant alarme (103).

9. Un appareil tel que revendiqué dans la revendication 8 dans lequel l'appareil comprend en outre une unité formant relais (113) distante de l'unité formant alarme (103) et de l'unité formant détecteur (112), l'unité formant détecteur (112) étant connectée en fonctionnement à l'unité formant relais (113), et l'unité formant relais (113) étant connectée en fonctionnement à l'unité formant alarme (103).

10. Un appareil tel que revendiqué dans la revendication 7 dans lequel l'unité formant détecteur (112) comprend cet au moins un détecteur de mouvement relatif, le module d'évaluation et au moins un moyen de communication.

11. Un appareil tel que revendiqué dans la revendication 8 dans lequel l'unité formant détecteur (112) comprend cet au moins un détecteur de mouvement relatif et au moins un moyen de communication, et l'unité formant alarme (103) comprend au moins un moyen de communication, le module d'évaluation et une alarme, le module d'évaluation étant connecté en fonctionnement à l'alarme.

12. Un appareil tel que revendiqué dans la revendication 9 dans lequel l'unité formant détecteur (112) comprend cet au moins un détecteur de mouvement relatif et au moins un moyen de communication, et l'unité formant relais (113) comprend au moins un moyen de communication et le module d'évaluation, et l'unité formant alarme (103) comprend au moins un moyen de communication et une alarme, cet au moins un moyen de communication dans l'unité formant alarme (103) étant connecté en fonctionnement à l'alarme.

13. Un appareil tel que revendiqué dans n'importe quelle revendication précédente, dans lequel le seuil d'activation prédéterminé est réglable.

14. Un appareil tel que revendiqué dans n'importe quelle revendication précédente, dans lequel cet au moins un détecteur de mouvement relatif est adapté pour détecter en outre un déplacement en rotation par rapport à l'axe le plus long du corps de l'individu.

15. Un appareil tel que revendiqué dans n'importe quelle revendication précédente, dans lequel le détecteur de mouvement relatif est adapté pour détecter en outre un déplacement vertical par rapport à l'axe le plus court du corps de l'individu.
